# EUROPEAN PATENT APPLICATION

(11) **EP 4 365 294 A1**
(43) Date of publication of application: **08.05.2024**
(21) Application number: 21945440.2
(22) Date of filing: 16.06.2021
(51) Int. Cl.: C12N 15/29, C12N 15/82, C07K 14/415

(54) **APPLICATION OF PLANT JAZ PROTEINS AND DERIVED POLYPEPTIDES IN PREVENTION AND TREATMENT OF HUMAN AND ANIMAL TUMORS**

(71) Applicant: Anyang Institution Of Sino-Cotton Industry Innovation, Anyang, Henan 455000 (CN)
(72) Inventor: LI, Fuguang, Anyang, Henan 455000 (CN); REN, Maozhi, Anyang, Henan 455000 (CN); XING, Yadi, Anyang, Henan 455000 (CN)
(74) Representative: Isarpatent
(86) International application number: PCT/CN2021/100319
(87) International publication number: WO 2022/261851

(57) **Abstract**

The present invention discloses the application of plant JAZ protein and its derived polypeptides in the prevention and treatment of tumors in human and animals. The present invention provides the application of plant JAZ protein or its derived polypeptides or modified products or related biomaterials in the preparation of products for the treatment and/or prevention of human or animal tumors, inhibition of the occurrence and development of human or animal tumors, inhibition of human or animal tumor cell proliferation, and promotion of human or animal tumor cell apoptosis. Cotton JAZ protein has a significant inhibitory effect on various human-derived tumor cell lines and has a significant inhibitory effect on the occurrence and development of tumors in mice *in vivo,* and JAZ protein has a strong interaction with human-derived oncogene MYCs. JAZ protein may target various types of human and animal tumors and can be developed as a targeting drug against human and animal tumors. The present invention provides novel drugs or adjunctive drugs for the treatment of various human and animal tumors.

## Description

### TECHNICAL FIELD

The present invention relates to the field of biotechnology, and specifically to the application of plant JAZ protein and its derived polypeptides in the prevention and treatment of tumors in human and animals.

### BACKGROUND

Plant callus culture is one of the key technical procedures in the breeding process by the gene editing technology. Plant callus is a physiological neoplasm that is produced during wound healing upon stress-induced damage in plants. It is a clump of amorphous parenchyma cells that have lost their original structures and functions, restored their abilities to divide, and could proliferate infinitely. Plants may produce "tumors" to heal wounds, and the rapid proliferation of cells is inhibited after wound healing without malignant proliferation. During the development of plant callus (plant tumor), JA signaling pathway is a key signaling pathway that regulates the development of plant "tumor" and promotes wound healing. The plant specific JAZ protein is the only negative regulator of the jasmonic acid signaling pathway and a key factor that prevents the development of "plant tumor". JAZ protein is a plant specific protein that is more common in cotton. It has been found that the growth of callus in cotton may be inhibited by overexpression of cotton JAZ during tissue culture. JA inhibitors may also inhibit the growth of cotton callus, and the inhibition of JA signaling by JAZ is achieved by inhibiting the transcription factor function of MYC.

"Plant tumor" is similar to human tumor, for example, in terms of morphology, production induced by exogenous stimuli, rapid cell division and proliferation, and regulation by the transcription factor MYC. The growth of "plant tumor" cannot develop to be malignant due to the strict regulation by JA signaling, whereas human tumor often deteriorates into cancer due to its uncontrollability. Cancer seriously affects human health, therefore, it is urgent to explore the mechanisms of cancer occurrence and develop cancer treatment strategies. The activation of the proto-oncogene MYC that is the most extensively studied oncogene is associated with the occurrence and development of various types of cancers. Given that MYC plays a critical role in the occurrence and development of cancer, screening drugs that target and inhibit MYC activity is an important strategy for treating malignant tumors. Currently, the most investigated drugs targeting MYC are those that inhibit the interaction of MYC with MAX, for example, small molecule inhibitors such as Mycro 3, SI-3716, MYCMI-6, sAJM589 and the like; polypeptide drug OmoMYC; and natural products such as Stauprimide, Lusianthridin and the like. Due to the complex structure composed of four helix beams may be formed between MYC and MAX, and the complex interaction between targets, drug development based on this structure is difficult, therefore, it is of great significance to find natural proteins that are easy to produce and can effectively inhibit MYC.

### SUMMARY OF THE INVENTION

The object of the present invention is to provide a novel drug for tumor prevention and treatment, namely JAZ protein, JAZ homologous protein and its derived polypeptides and the like, which has important drug application value in tumor prevention and treatment.

The technical problem to be solved by the present invention is to overcome the difficulties in drug development based on the complex interaction between proto-oncogenes MYCs (c-Myc, NMYC, LMYC) targets in human and animal tumors, and aims to explore drugs for preventing and treating tumors against the long-term harm of tumors to human and animals.

The present invention proposes a natural product based on a multi-target anti-tumor mechanism, which solves the problems such as single target, drug resistance, and low efficiency of traditional tumor therapeutic drugs.

In a first aspect, the present invention claims an application of a), b), c) or d) in any one of the following P1-P4:
a). a plant JAZ protein.
b). a polypeptide derived from the plant JAZ protein.
c). a modified product of the plant JAZ protein in a) or the derived polypeptide in b); the modified product may be a product obtained by modifying the plant JAZ protein or the derived polypeptide after any one of the following modifications: glycosylation, phosphorylation, N-methylation, myristoylation, palmitoylation, biotinylation, fluorescence labeling, polyethylene glycol (PEG) modification, multiple antigen peptide (MAP), isoprene cyclization, cyclization, and the like.
d). a relevant biomaterial of the plant JAZ protein in a) or the derived polypeptide in b); the relevant biomaterial is any one of the followings: 1) a nucleic acid molecule encoding the plant JAZ protein or the derived polypeptides; 2) an expression cassette, an expression vector, or a recombinant microorganism containing the nucleic acid molecule.

P1. Preparation of products for the treatment and/or prevention of tumors in human or animals.

P2. Preparation of products for inhibiting the occurrence and/or development of tumors in human or animals.

P3. Preparation of products for inhibiting the proliferation of tumor cells in human or animals.

P4. Preparation of products for promoting apoptosis of tumor cells in human or animals.

In this application, the plant JAZ protein can interact with MYC protein of humans or animals.

Furthermore, the MYC protein may be c-Myc protein, NMYC protein, and/or LMYC protein. Specifically, the plant JAZ protein may interact with the N-terminus of c-Myc protein relying on NT domain.

In this application, the plant JAZ protein has all or some of the following functions: blocking the cell cycle of a human or animal tumor cell at G2-M phase; regulating a gene downstream of c-Myc in human or animal tumor cells; interacting with uPAR, a surface receptor for cancer cells (the plant JAZ protein may interact with D3 domain of uPAR relying on NT domain ); interacting with uPA, a ligand for the surface receptor uPAR for cancer cells (interacting with ATF domain of uPA); and entering into the nucleus of human or animal tumor cells.

In the present invention, the regulation of a gene downstream of c-Myc is specifically embodied as all or some of the followings: apoptosis-related genes downstream of c-Myc, Bcl2, BAX, HK2, FASN, and MCM5 are down-regulated, and the P21 gene is up-regulated; and cell-cycle-related genes, CCNA2, CCNB1, and CCND1 are down-regulated, and CCNE1 is up-regulated.

Furthermore, the polypeptides derived from plant JAZ protein may be or contain NT domain, ZIM domain, or Jas domain of the plant JAZ protein.

In the specific embodiments of the present invention, the plant JAZ protein is specifically the JAZ protein derived from cotton.

Furthermore, the JAZ protein derived from cotton may be any one of the followings:
(A1) a protein having an amino acid sequence as set forth in any one of SEQ ID No.1 to SEQ ID No.35;
(A2) a protein obtained by substitution and/or deletion and/or addition of one or more amino acid residues in the amino acid sequence defined in (A1) and derived from cotton having the same function;
(A3) a protein having more than 99%, more than 95%, more than 90%, more than 85%, or more than 80% identity to the amino acid sequence defined in any one of (A1)-(A2) and derived from cotton having the same function; and
(A4) a fusion protein obtained by linking a protein tag at the N-terminus and/or C-terminus of the protein defined in any one of (A1)-(A3).

Of the above proteins, the protein-tag refers to a polypeptide or protein that is fused and expressed with a protein of interest using DNA recombination technology *in vitro* to facilitate the expression, detection, tracing, and/or purification of the protein of interest. The protein-tag may be a Flag tag, His tag, MBP tag, HA tag, myc tag, GST tag, and/or SUMO tag, etc.

Of the above proteins, the identity refers to that to the amino acid sequence. The identity to the amino acid sequence may be determined using the homology search site on the international internet, such as BLAST webpage on the NCBI homepage website. For example, the value of identity (%) may be obtained by retrieving the identity of a pair of amino acid sequences for calculation in the advanced BLAST2.1, using blast program, setting the Expect value to 10, all Filters to OFF, using BLOSUM62 as Matrix, setting Gap existence cost, Per residual gap cost, and Lambda ratio to 11, 1, and 0.85(default values), respectively.

Of the above proteins, the homology of more than 95% may be at least 96%, 97%, and 98% identity. The homology of more than 90% may be at least 91%, 92%, 93%, and 94% identity. The homology of more than 85% may be at least 86%, 87%, 88%, and 89% identity. The homology of more than 80% may be at least 81%, 82%, 83%, and 84% identity.

Correspondingly, the nucleic acid molecule encoding the plant JAZ protein (i.e. the JAZ protein derived from cotton) may be any one of the followings:
(B1) a DNA molecule as set forth in any one of SEQ ID No.36 to SEQ ID No.70;
(B2) a DNA molecule that hybridizes with the DNA molecule defined in (B1) under a strict condition and encodes the JAZ protein derived from cotton;
(B3) a DNA molecule having more than 99%, more than 95%, more than 90%, more than 85%, or more than 80% homology to the DNA sequence defined in any one of (B1)-(B2) and encoding the JAZ protein derived from cotton.

The DNA molecules set forth in SEQ ID No.36 to SEQ ID No.70 sequentially encode the proteins set forth in SEQ ID No.1 to SEQ ID No.35.

Of the above nucleic acid molecules, the strict condition may be the followings: hybridizing in a mixed solution of 7% sodium dodecyl sulfate (SDS), 0.5 M Na₃PO₄, and 1 mM EDTA at 50°C, followed by rinsing in 2 × SSC and 0.1% SDS at 50°C; alternatively, hybridizing in a mixed solution of 7% SDS, 0.5 M Na₃PO₄, and 1 mM EDTA at 50°C, followed by rinsing in 1 × SSC and 0.1% SDS at 50°C; alternatively, hybridizing in a mixed solution of 7% SDS, 0.5 M Na₃PO₄, and 1 mM EDTA at 50°C, followed by rinsing in 0.5 × SSC and 0.1% SDS at 50°C; alternatively, hybridizing in a mixed solution of 7% SDS, 0.5 M Na₃PO₄, and 1 mM EDTA at 50°C, followed by rinsing in 0.1 × SSC and 0.1% SDS at 50°C; alternatively, hybridizing in a mixed solution of 7% SDS, 0.5 M Na₃PO₄, and 1 mM EDTA at 50°C, followed by rinsing in 0.1 × SSC and 0.1% SDS at 65°C; alternatively, hybridizing in a solution of 6 × SSC and 0.5% SDS at 65°C, followed by washing the membrane once each in 2 × SSC and 0.1% SDS and 1 × SSC and 0.1% SDS.

Of the above nucleic acid molecules, the homology refers to the identity to the nucleotide sequence. The identity to the nucleotide sequence may be determined using the homology search site on the international internet, such as BLAST webpage on the NCBI homepage website. For example, the value of identity (%) may be obtained by retrieving the identity of a pair of nucleotide sequences for calculation in the advanced BLAST2.1, using blast program, setting the Expect value to 10, all Filters to OFF, using BLOSUM62 as Matrix, and setting Gap existence cost, Per residual gap cost, and Lambda ratio to 11, 1, and 0.85(default values), respectively.

Of the above proteins, the homology of more than 95% may be at least 96%, 97%, and 98% identity. The homology of more than 90% may be at least 91%, 92%, 93%, and 94% identity. The homology of more than 85% may be at least 86%, 87%, 88%, and 89% identity. The homology of more than 80% may be at least 81%, 82%, 83%, and 84% identity.

Furthermore, the human or animal tumors include but are not limited to ovarian, breast, cervical, lung cancers and the like.

Furthermore, the human or animal tumor cells include but are not limited to ovarian, breast, cervical, lung cancer cells and the like.

In specific embodiments of the present invention, the ovarian cancer cells are SKOV3 cells; the breast cancer cells are MCF7 cells; the cervical cancer cells are Hela cells; and the lung cancer cells are A549 cells.

In the above applications, the product may be a drug.

Furthermore, the drug is prepared by addition of pharmaceutically acceptable adjuvants (such as excipients, diluents and the like) on the basis of a), b), or c).

Furthermore, the drug may also be a compound drug or an adjuvant drug.

Still furthermore, the drug may be in a form suitable for oral administration such as a tablet, capsule, granule, or syrup, etc., or in a form suitable for injection administration such as a powder or solution for injection.

In a second aspect, the present invention claims the application of the a), b), c) or d) described in the preceding first aspect in inhibiting the growth of plant callus.

Furthermore, the plant callus is cotton callus.

In specific embodiments of the present invention, the explant is a root segment or hypocotyl.

In this application, the plant JAZ protein has all or some of the following functions: interacting with NINJA protein, TPL protein, and HDA6 protein in the plant; interacting with COI1 protein in the presence of JA-Ile; and being capable of forming complexes with MYC2 protein, NINJA protein, and TPL protein.

In specific embodiments of the present invention, the plant is cotton.

In a third aspect, the present invention claims the application of a JA synthesis inhibitor in inhibiting the growth of plant callus.

In specific embodiments of the present invention, the JA synthesis inhibitor is DICEA that is a copper reagent or ibuprofen.

Furthermore, the plant callus is cotton callus.

In specific embodiments of the present invention, the explant is a root segment or hypocotyl.

In a fourth aspect, the present invention claims a method for inhibiting the growth of plant callus.

The method for inhibiting the growth of plant callus, as claimed in the present invention, is to inhibit the growth of plant callus by the a), b), c) or d) described in the preceding first aspect or the JA synthesis inhibitor described in the preceding third aspect.

In specific embodiments of the present invention, the plant callus is cotton callus.

Furthermore, the method may include the following steps of:
the experimental group: overexpressing the plant JAZ protein or the derived polypeptides described in the preceding first aspect in the plant of interest to obtain a transgenic positive plant, taking an explant from the transgenic positive plant for tissue culture to obtain a callus;
the control group: the only difference from the experimental group is that the explant taken for tissue culture is from the plant of interest that is not transgenic; and
the growth rate of the callus in the experimental group is slower than that in the control group.

Alternatively,
the method may further include the following steps of:
the experimental group: placing an explant taken from a plant of interest in the callus induction medium supplemented with the JA synthesis inhibitor for tissue culture to obtain a callus;
the control group: the only difference from the experimental group is that the callus induction medium for tissue culture does not contain the JA synthesis inhibitor; and
the growth rate of the callus in the experimental group is slower than that in the control group.

In specific embodiments of the present invention, the explant is a root segment or hypocotyl.

The JA synthesis inhibitor may have a final concentration of 200-800 µM, such as 200 µM, 400 µM or 800 µM in the callus induction medium in the case that the JA synthesis inhibitor is copper reagent DICEA; and may have a final concentration of 20-80 µM, such as 20 µM, 40 µM or 80 µM in the callus induction medium in the case that the JA synthesis inhibitor is ibuprofen.

In a fifth aspect, the present invention claims a method for treating and/or preventing human or animal tumors.

The method for treating and/or preventing human or animal tumors as claimed in the present invention is to treat and/or prevent human or animal tumors using a drug with the a), b) or c) described in the preceding first aspect above as an active ingredient.

In a sixth aspect, the present invention claims a method for inhibiting the occurrence and/or development of human or animal tumors.

The method for inhibiting the occurrence and/or development of human or animal tumors as claimed in the present invention is to inhibit the occurrence and/or development of human or animal tumors using a drug with the a), b) or c) described in the preceding first aspect as an active ingredient.

In the fifth and sixth aspects, the human or animal tumors include but are not limited to ovarian, breast, cervical, lung cancers and the like.

In a seventh aspect, the present invention claims a method for inhibiting the proliferation of human or animal tumor cells.

The method for inhibiting the proliferation of human or animal tumor cells as claimed in the present invention is to inhibit the proliferation of human or animal tumor cells by the a), b) or c) described in the preceding first aspect.

In an eighth aspect, the present invention claims a method for promoting apoptosis of human or animal tumor cells.

The method for promoting apoptosis of human or animal tumor cells as claimed in the present invention is to promote apoptosis of human or animal tumor cells by the a), b) or c) described in the preceding first aspect.

In the seventh and eighth aspects, the concentration of the a) or b) or c) applied at a cellular level should be controlled at more than 1 µM and less than 10 µM.

In the seventh and eighth aspects, the human or animal tumor cells include but are not limited to ovarian, breast, cervical, lung cancer cells and the like.

In specific embodiments of the present invention, the ovarian cancer cells are SKOV3 cells; the breast cancer cells are MCF7 cells; the cervical cancer cells are Hela cells; and the lung cancer cells are A549 cells.

It has been found that the maximum non-toxic concentration of the JAZ protein, JAZ homologous proteins, and their derived polypeptides to the organic cells are 10 µM through research in the invention. Therefore, in practical applications, the concentration of the JAZ protein, JAZ homologous proteins, and their derived polypeptides applied at a cellular level should be controlled at less than 10 µM in order to avoid cytotoxicity.

The present invention has discovered a new function of JAZ protein through extensive researches, which may inhibit various human and animal cancer cells. The JAZ protein inhibits MCF7 cells with IC50=0.691µM, inhibits A549 cells with IC50 = 0.697µM, inhibits Hela cells with IC50=0.553 µM, and inhibits SKOV3 cells with IC50 = 0.784 µM at a cellular level.

In a ninth aspect, the present invention claims a drug having or containing the a), b), or c) described in the preceding first aspect as an active ingredient, which may be used for any one of the followings: treatment and/or prevention of human or animal tumors, inhibition of the occurrence and/or development of human or animal tumors, inhibition of the proliferation of human or animal tumor cells, and promotion of apoptosis of human or animal tumor cells.

Furthermore, the human or animal tumors include but are not limited to ovarian, breast, cervical, lung cancers and the like.

Furthermore, the human or animal tumor cells include but are not limited to ovarian, breast, cervical, lung cancer cells and the like.

In specific embodiments of the present invention, the ovarian cancer cells are SKOV3 cells; the breast cancer cells are MCF7 cells; the cervical cancer cells are Hela cells; and the lung cancer cells are A549 cells.

In a tenth aspect, the present invention claims a preparation having or containing the a), b), or c) described in the preceding first aspect or the JA synthesis inhibitor described in the preceding third aspect as an active ingredient, which is used to inhibit the growth of plant callus.

Furthermore, the plant callus is cotton callus.

In specific embodiments of the present invention, the explant is a root segment or hypocotyl.

In the present invention, treating the human or animal tumors is preferably treating the human or animal tumors in the early or recovery period.

Since cotton has been traditionally used as a traditional Chinese medicine since ancient times (as recorded in "Compendium of Materia Medica", etc.), and there is a large amount of JAZs in cotton. The plant specific JAZ protein may block the activity of transcription factors such as MYC in plants, while MYC is highly conserved in evolution. Accordingly, the present invention proposes that the cotton JAZ protein, JAZ homologous proteins and their derived polypeptides, as well as modified products of the above proteins and polypeptides, inhibit human and animal tumors by inhibiting human and animal proto-oncogenes MYCs (c-Myc, NMYC and LMYC), in order to achieve the goal of preventing and treating tumors.

### BRIEF DESCRIPTION OF DRAWINGS

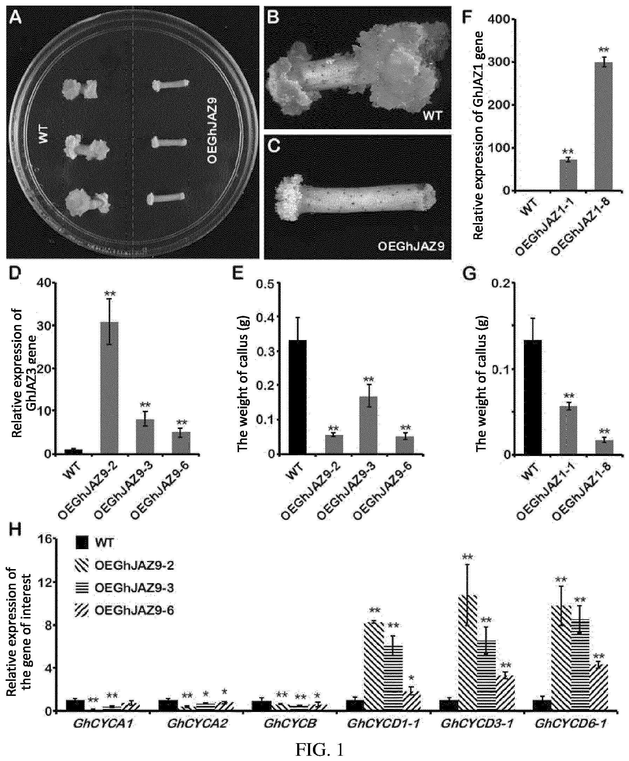

FIG. 1 shows that overexpression of GhJAZs inhibits the growth of cotton callus. A. Calluses of "CCRI 24" (WT) and "CCRI 24" in which GhJAZ9 is overexpressed (control WT on the left and the material in which GhJAZ9 is overexpressed on the right), bar = 1 cm; B. Enlarged view of callus of wild-type "CCRI 24" , bar = 0.3 cm; C. Enlarged view of callus in which GhJAZ9 is overexpressed, bar = 0.3 cm; D. Analysis of gene expression of GhJAZ9 gene in wild-type and overexpression transgenic strains; E. Statistics on the weights of calluses of wild-type and overexpression transgenic strains; F. Analysis of gene expression of GhJAZ1 gene in wild-type and overexpressed rooting cotton; G. Statistics on the weights of root calluses of wild-type and overexpressed rooting cotton; H. Analysis of expression of cell cycle genes in calluses of wild-type and overexpressed transgenic strains.

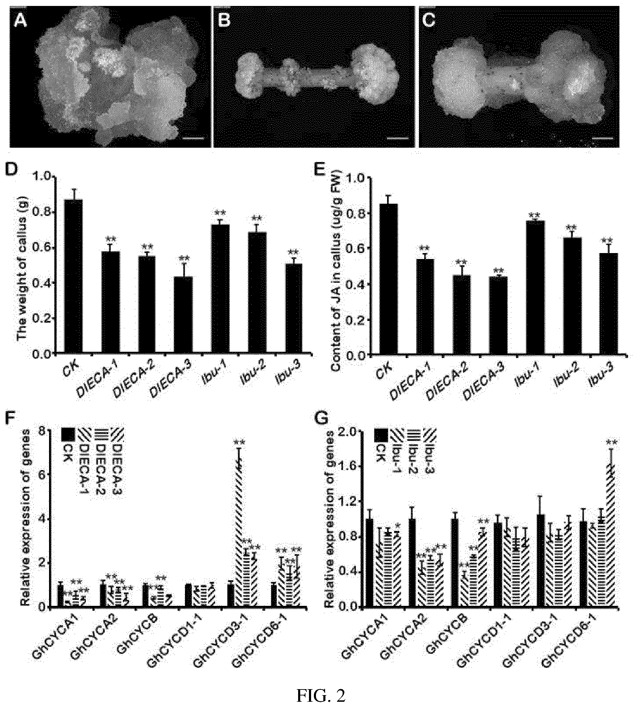

FIG. 2 shows that addition of an exogenous JA synthesis inhibitor may significantly inhibit the growth of cotton callus. A. Callus of "CCRI 24" grown on the normal medium for 5 weeks; B. Callus of "CCRI 24" grown on the medium containing DIECA for 5 weeks; C. Callus of CCRI 24 grown on medium containing ibuprofen for 5 weeks; D. Statistics on the weights of callus grown for 5 weeks (Ibu represents ibuprofen); E. Detection of JA content in callus (Ibu represents ibuprofen); F. Expression of cell cycle genes in callus on the medium containing DIECA; G. Validation of expression of cell cycle genes in callus on the medium containing ibuprofen. Bar = 0.2 cm. In the figure, DIECA-1, DIECA-2, and DIECA-3 represent three groups supplemented with DIECA at a concentration of 200 µM, 400 µM and 800 µM in the callus induction medium, respectively; Ibu-1, Ibu-2, and Ibu-3 represent three groups supplemented with Ibu at a concentration of 20 µM, 40 µM and 80 µM in the callus induction medium, respectively.

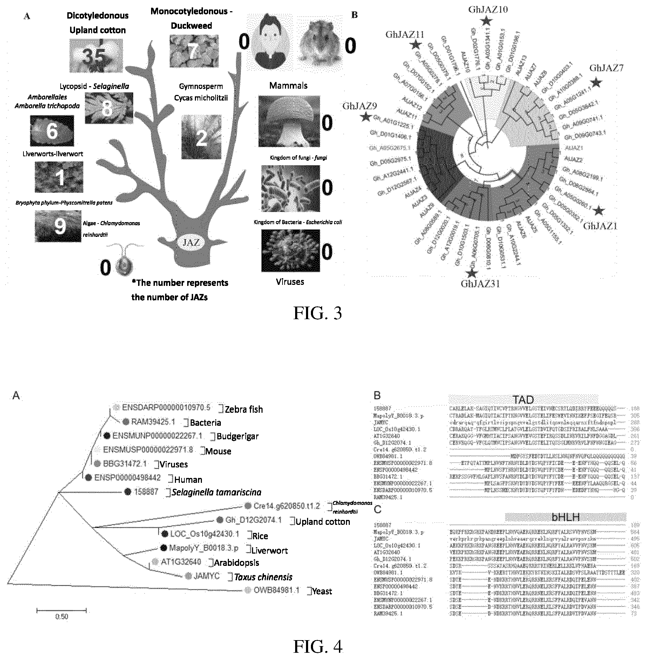

FIG. 3 shows the distribution and phylogenetic tree of JAZ gene in various organisms. A. Evolutionary distribution of JAZ protein in the biosphere; B. Phylogenetic analysis of JAZ protein in Arabidopsis and cotton.
FIG. 4 shows analysis of evolutionary and conserved domain of MYC in the biosphere. A. Evolutionary distribution of MYC protein in the biosphere; B and C. Analysis of conserved domain of MYC protein.

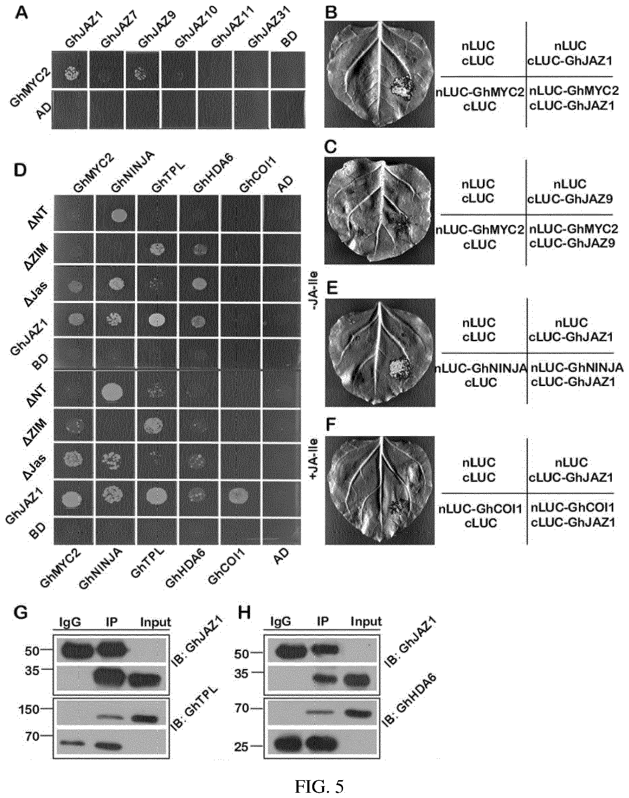

FIG. 5 shows the interaction of major proteins in the cotton JAZ protein transcriptional inhibition complex with JAZ. A. Screening by yeast two hybrid for the interaction of cotton JAZ with MYC2(AD represents pGADT7 mock for yeast two hybrid; BD represents pGBKT7 mock for yeast two hybrid, which is equivalent to a negative control) ; B. Validation of the interaction of GhJAZ1 with GhMYC2 by luciferase experiment; C. Validation of the interaction of GhJAZ9 with GhMYC2 by luciferase experiment; D. Validation of the interaction of GhJAZ1 and JAZ1 mutants of NT, ZIM, and Jas deficient, respectively (those marked with ΔNT, ΔZIM and ΔJas in the figure represent deletion of corresponding domains, respectively) with GhMYC2, GhNINJA, GhTPL, GhHDA6, and GhCOI1(with no JA-Ile in the medium on the top, and JA-Ile added in the medium on the bottom). E. Validation of the interaction of GhJAZ1 with GhNINJA by luciferase experiment; F. Validation of the interaction of GhJAZ1 with GhCOI1 by luciferase experiment; G. Validation of the interaction of GhJAZ1 with GhTPL by CO-IP experiment; H. Validation of the interaction of GhJAZ1 with GhHDA6 by CO-IP experiment.

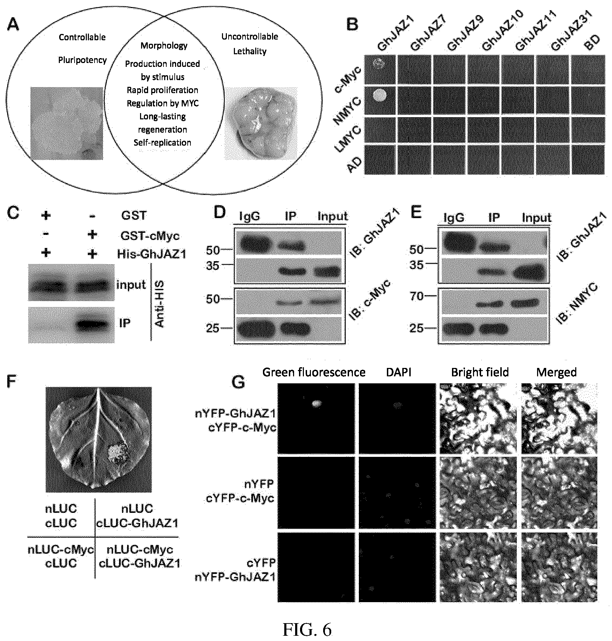

FIG. 6 shows the direct interaction of cotton JAZ with the proto-oncogene MYC. A. Similarities between plant "tumors" and animal tumors; B. Screening by yeast two hybrid for the interaction of cotton JAZ with the proto-oncogene MYC (AD represents pGADT7 mock for yeast two hybrid; BD represents pGBKT7 mock for yeast two hybrid, which is equivalent to a negative control); C. Validation of the interaction of GhJAZ1 with c-Myc by pull down experiment; D. Validation of the interaction of GhJAZ1 with c-Myc by Co-IP; E. Validation of the interaction of GhJAZ1 with NMyc by Co-IP; F. Validation of the interaction of GhJAZ1 with c-Myc by LUC experiment; G. Validation of the interaction of GhJAZ1 with c-Myc by double molecule fluorescence complementary. Bar = 50 µm.

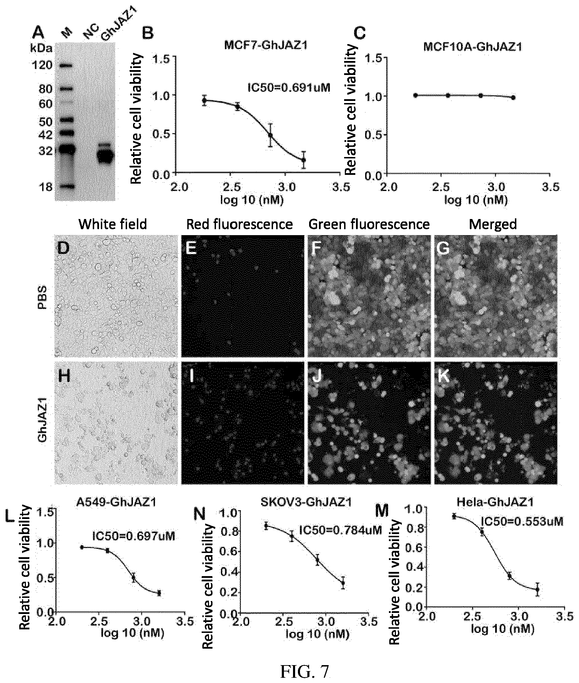

FIG. 7 shows inhibition of the proliferation of cancer cells by GhJAZ1 protein. A. Expression of GhJAZ1-His protein detected by WB; B. Effect of GhJAZ1 on MCF7 cells, IC50 = 0.691 µM; C. Effect of GhJAZ1 on MCF10A cells, without inhibitory effect; D-G. PBS-treated breast cancer cell MCF7; H-K. GhJAZ1 protein-treated breast cancer cell MCF7(D and H: images taken in the bright field; E and I: images taken under red fluorescence, apoptotic cells are shown in red; F and J, images taken under green fluorescence, live cells are shown in green; G and K: results of merged images under red and green fluorescences); L. Effect of GhJAZ1 protein on A549 cells, IC50 = 0.697 µM; M. Effect of GhJAZ1 protein on Hela cells, IC50 = 0.553 µM; N. Effect of GhJAZ1 protein on SKOV3 cells, IC50 = 0.784 µM. Bar = 100 µm.

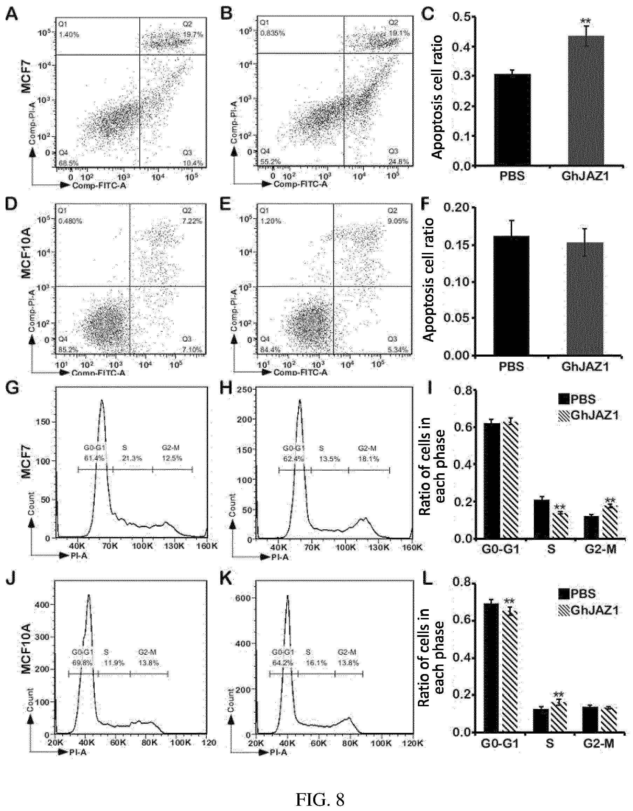

FIG. 8 shows the effect of GhJAZ1 protein on apoptosis and cell cycle of cancer cells and normal cells. A-C. Effect of GhJAZ1 protein on apoptosis of MCF7 cells; D-F. Effect of GhJAZ1 protein on apoptosis of MCF10A cells; G-I. Effect of GhJAZ1 protein on cell cycle of MCF7 cells; J-L. Effect of GhJAZ1 protein on cell cycle of MCF10A cells. Wherein A, D, G, and J represent control groups (with PBS added), B, E, H, and K represent GhJAZ1 treated groups, and C, F, I, and L are shown as statistical data.

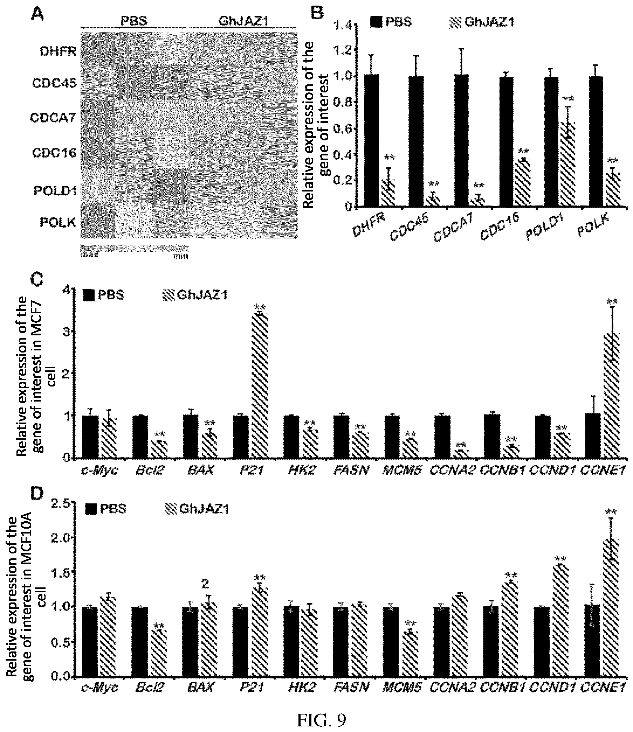

FIG. 9 shows the regulation of c-Myc downstream genes by GhJAZ1. A. Differential gene heatmap analysis of transcriptome in MCF7 cells treated with PBS and GhJAZ1 protein; B. Detection of differential gene expression in MCF7 cells treated with PBS and GhJAZ1 protein; C. Detection of c-Myc downstream genes expression in MCF7 cells treated with PBS and GhJAZ1 protein; D. Detection of c-Myc downstream genes expression in MCF10A cells treated with PBS and GhJAZ1 protein.

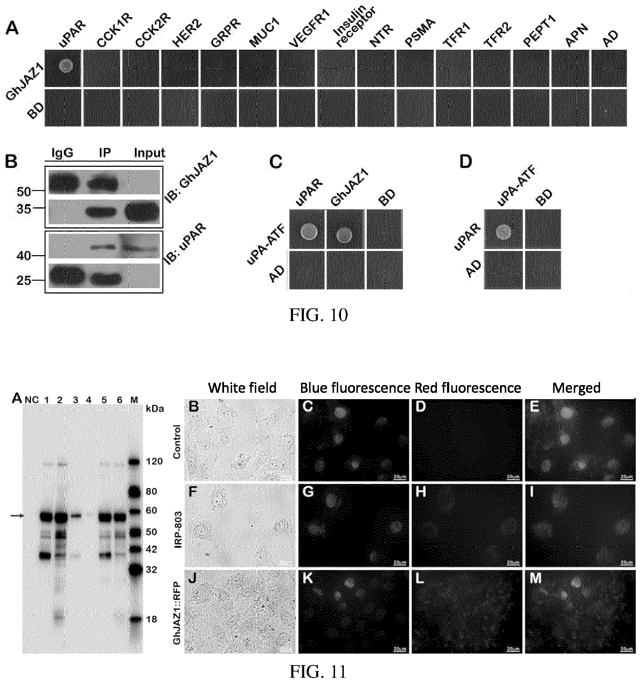

FIG. 10 shows the direct interaction of GhJAZ1 with the surface receptors for cancer cells. A. Screening by yeast two hybrid for the interaction of GhJAZ1 with the surfacer eceptors for cancer cells (AD represents pGADT7 mock for yeast two hybrid; BD represents pGBKT7 mock for yeast two hybrid, which is equivalent to a negative control); B. Validation of the interaction of GhJAZ1 with uPAR, the surface receptor for cancer cells by CO-IP; C and D. Analysis of the interaction of uPAR and GhJAZ1 with the ATF domain of uPA, respectively.
FIG. 11 shows the experimental validation of the entry of GhJAZ1 protein into cells and nuclei. A. WB detection of GhJAZ1+RFP-His protein. Lane M: the marker for Western Blotting; Lane NC: the cell lysate without induction; Lane 1: the cell lysate induced at 15°C for 16 hours; Lane 2: the cell lysate induced at 37°C for 4 hours; Lane 3: the supernatant of cell lysate induced at 15°C for 16 hours; Lane 4: the supernatant of cell lysate induced at 37°C for 4 hours; Lane 5: fragments of cell lysate induced at 15°C for 16 hours; Lane 6: fragments of cell lysate induced at 37°C for 4 hours. Anti-histidine antibody (GenScript, Cat. No. A00186) is used as the primary antibody for immunoblotting; B-E. PBS-treated breast cancer cell MCF7; F-I. IRP-803(uPAR inhibitor)-treated breast cancer cells MCF7; J-M. GhJAZ1 protein-treated breast cancer cells MCF7(B, F and J: images taken under the bright field; C, G and K: images taken under blue fluorescence, the nucleus are shown in blue; D. H and L: images taken under red fluorescence, wherein H shows red fluorescence from IRP-803 outside the cell nucleus, and L shows red fluorescence from GhJAZ1+GFP distributed in a dotted pattern; E, I and M: results of merged images under blue and red fluorescences).

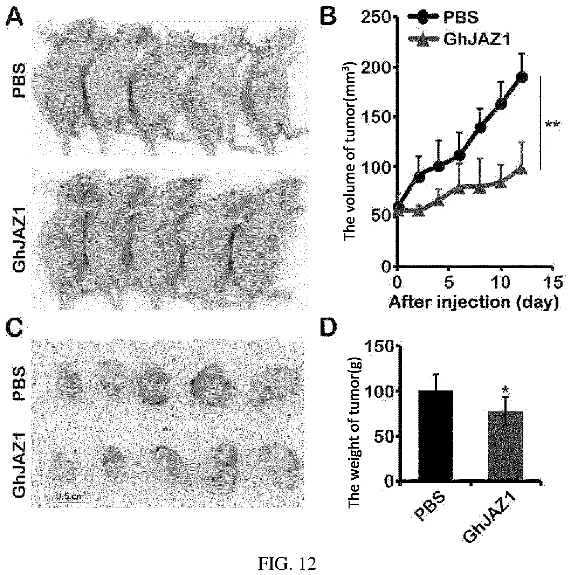

FIG. 12 shows the results of anti-tumor experiment of GhJAZ1 protein *in vivo.* A. Images of tumors *in vivo* in the dosing and control groups; B. Statistics of tumor volumes in the dosing and control groups; C. Images of tumors *in vivo* in the dosing and control groups; D. Statistics on the weights of tumors in the dosing and control groups.

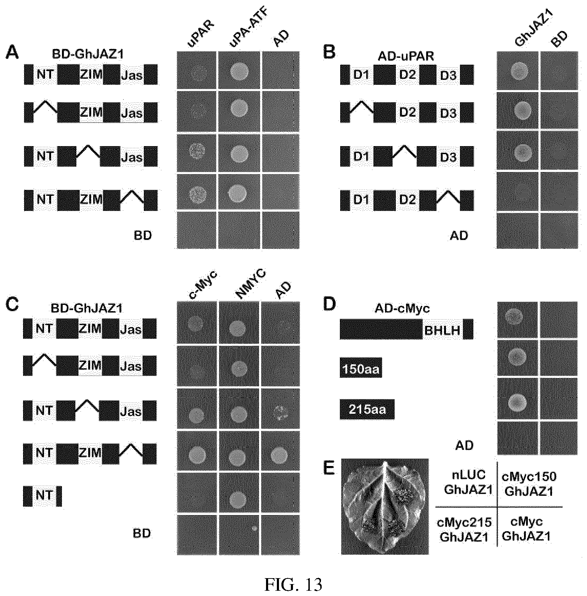

FIG. 13 shows the results of experiments on the interaction domains of GhJAZ1 with uPAR and c-Myc. A. Analysis of the interaction of yeast vectors for NT, ZIM, and JAS domain deficient GhJAZ1 and GhJAZ1 with uPAR and ATF domain of uPA by yeast two hybrid, respectively; B. Analysis of the interaction of yeast vectors for uPAR and D1, D2, and D3 domain deficient uPAR with GhJAZ1, respectively; C. Analysis of the interaction of yeast vectors for NT, ZIM, and JAS domain deficient GhJAZ1 and GhJAZ1 with the full length of c-Myc and NMYC, respectively; D. Analysis of the interaction of c-Myc and the truncated first 150 and 215 amino acids of c-Myc with GhJAZ1, respectively; E. Validation of the interaction of c-Myc and the truncated first 150 and 215 amino acids of c-Myc with GhJAZ1 by LUC, respectively.

### DETAILED DESCRIPTION OF EMBODIMENTS

The Examples below facilitate a better understanding of the present invention, but do not limit the present invention. The experimental methods in Examples below are conventional methods unless otherwise specified. The experimental materials used in Examples below were all conventional commercially available biochemical reagents, unless otherwise specified. Mean values were used for results from the quantitative experiments in triplicate in Examples below.

### Example 1. Analysis of effect of cotton JAZ overexpression on the growth of callus

### 1. Creation of transgenic materials overexpressing GhJAZ1

Full-length and domain conservation analysis was performed based on the amino acid sequence of Arabidopsis JAZ1 in the cotton database, in order to identify the cotton JAZ1 with the highest homology to AtJAZ1, with the gene number of Gh_A05G0260 and having the sequence set forth in SEQ ID No.4. The full-length CDS of GhJAZ1(SEQ ID No.39) was ligated between the modified pCAMBIA2300(the original vector of pCAMBIA2300 is a product commercially available from Youbio, Cat. No.: VT1383, the specific modification is as follows: replace the MCS region of the original vector of pCAMBIA2300 with the sequence of 2X35S-YFP, and the sequence of 2X35S-YFP is as shown in SEQ ID No. 99.) XbaI and PacI cleavage sites, and the recombinant positive plasmid obtained after sequencing verification was transformed into Agrobacterium K599. Utilizing *Agrobacterium rhizogenes* mediated genetic transformation technology, the rooting transgenic cotton materials were created by Agrobacterium K599 transformed with the recombinant positive plasmid using cotton "CCRI 24" as materials. Sequentially, sterile seedlings were cultured, followed by preparation of cotyledons, which were then immersed in Agrobacterium solution, and then the roots were grown out. The obtained resistant roots were identified at the RNA level.

Identification at the RNA level: RNA was extracted using RNA extraction kit (RNAprep Pure Polysaccharide Polyphenol Plant Total RNA Extraction Kit (DP441), manufacturer: TIANGEN BIOTECH (BEIJING) CO.,LTD.), and then cDNA was obtained using two-step reverse transcription kit (a third-generation premix for two-step de-genomization and reverse transcription, Cat. No.: MR05201). cDNA as a template was added into a 96-well plate, with addition of SYBR^{®}Green I chimeric dye (an antibody dye quantitative PCR premix, Cat. No.: MQ10101) and gene primers for three-step qPCR amplification. Finally, the relative gene expression was calculated based on the Ct value.

Actin was used as the internal reference and its detection primers were;
Q-Actin-F: 5' -GACCCAGATCATGTTTGAGACCT-3' ;
Q-Actin-R: 5'-CAGTGTGGCTGACACCATCAC-3'.

Primers for detecting GhJAZ1 were:
Q-GhJAZ1-F: 5'-GATGATAGTCCCAACAAGTTGGAGCC-3';
Q-GhJAZ1-R: 5'-TCGCTTCTCTAGGAACCGATGCA-3'.

The results showed that OEGhJAZ1-1 and OEGhJAZ1-8 were highly significantly increased by 70-300 times compared to the control (F in FIG. 1). Wherein OEGhJAZ1-1 and OEGhJAZ1-8 were two positive strains randomly selected from transgenic offsprings.

### 2. Creation of transgenic materials overexpressing GhJAZ9

Full-length and domain conservation analysis was performed based on the amino acid sequence of Arabidopsis JAZ9 in the cotton database to identify the cotton JAZ9 having the sequence as set forth in SEQ ID No.2, which had the highest homology to AtJAZ9. GhJAZ9 full-length CDS (SEQ ID No.37) was ligated between EcoRI and EcoRI of vector pCR^{®}8/GW/TOPO (invitrogen, Cat. No.: K252020), followed by ligation with pEarlyGate 303 vector (YouBio, Cat. No.: VT9027) by LR reaction (ThermoFisher, Gateway LR Clonase Enzyme mix, Cat. No.: 11791019), and the recombinant positive plasmid after sequencing verification was transformed into *Agrobacterium tumefaciens* LBA4404, to create transgenic material by Agrobacterium-mediated genetic transformation using cotton "CCRI 24" as the material. Sequentially, sterile seedlings were cultured, followed by preparation of cotton explants, which were then immersed in Agrobacterium solution, and then resistant callus were formed after dedifferentiation, followed by regeneration into seedlings, and finally, resistant plants were obtained. The obtained resistant plants were identified at the RNA level. The method of identification at the RNA level was the same as above, and primers for detection GhJAZ9 were:
Q-CO39-F1: 5'-CTTCATGCCTATATCAACAATGGATGCT-3';
Q-CO39-R1: 5'-CAAGGGTGGGAAGAACTGAATGTG-3'.

The results showed that the expression levels of OEGhJAZ9-2, OEGhJAZ9JAZ9-3, and OEGhJAZ9-6 were highly significantly increased compared to the control (D in FIG. 1), wherein OEGhJAZ9-2, OEGhJAZ9-3, and OEGhJAZ9-6 were three positive strains randomly selected from transgenic offsprings.

### 3. Effect of cotton JAZ overexpression on the growth of callus

The GhJAZ1 overexpression positive roots and negative roots (i.e., the roots of CCRI 24 transfected with pCAMBIA2300 mock) identified in step 1 were cut off to form explants with consistent length and placed in a same petri dish for simultaneous cultivation. After one month, the growth of callus was observed and photographed with a camera and microscope, and statistics on the weights of callus were performed. Samples were taken for fluorescence quantitative PCR to detect the expression of cell cycle-related genes.

The steps of RNA extraction, reverse transcription, and fluorescence quantitative PCR were the same as above, and the sequences of primers were as follows:
Primers for detecting GhCYCA1:
   Q-GhCYCA1-1-F: 5'-GTACAACCCGGTTCAGTTCCG-3';
   Q-GhCYCA1-1-R: 5'-GAATCAACAGCCGTAACATCATG-3' .
Primers for detecting GhCYCA2:
   Q-GhCYCA2-4-F: 5' -GCACTTAATGGTCGAATCACACGT-3' ;
   Q-GhCYCA2-4-R: 5' -ATTATTGCAGCAAACATTTGTGACAT-3' .
Primers for detecting GhCYCB:
   Q-GhCYCB-F: 5'-GGCTCAGATTCAGACTCATCGTC-3';
   Q-GhCYCB -R: 5'-CAGCAGGAGCAGCAACACTTC-3'.
Primers for detecting GhCYCD1-1:
   Q-GhCYCD1-1-F: 5'-CTTCACGGACTTACTCTGCGC-3';
   Q-GhCYCD1-1-R: 5'-CACTGAAACCGAGCAAGGTAATC-3'.
Primers for detecting GhCYCD3-1:
   Q-GhCYCD3-1-F: 5'-ATGGCAATACAGCAATATGAACAGC-3';
   Q-GhCYCD3-1-R: 5'-CCTGCTCTAACAACAACAGTGGG-3'.
Primers for detecting GhCYCD6-1:
   Q-GhCYCD6-1-F: 5'-ACCCATTAACAAACTTCGATGACTT-3' ;
   Q-GhCYCD6-1-R: 5'-TCGAGATAATTGACAGCAAGATACG-3'.

Sterile seedlings were cultured using seeds of homozygous transgenic strains overexpressing GhJAZ9 identified in step 2 and of "CCRI 24", respectively, and hypocotyls were taken and cut off for callus cultvation on the callus induction medium (without hormones) after 7 days. Explants from strains overexpressing GhJAZ9 and from "CCRI 24" were cut to consistent length and placed in a same petri dish for simultaneous cultvation. After one month, the growth of callus was observed and photographed with a camera and microscope, and statistics on the weights of callus were performed. At the same time, samples were taken from callus overexpressing GhJAZ9 for fluorescence quantitative PCR to detect the expression of cell cycle-related gene (the steps of method and primers were the same as above).

The results showed that overexpression of JAZ in cotton resulted in slow growth of callus, and the expression of cell cycle regulatory genes of callus cells was severely affected (FIG. 1), indicating that overexpression of cotton JAZ could inhibit the growth of callus of cotton.

### Example 2. Analysis of effect of JA synthesis inhibitor on the growth of callus

JA signaling pathway might also be a key signaling pathway in regulating callus formation in cotton. It had been reported internationally that there were JA synthesis inhibitors such as DICEA that a copper reagent and ibuprofen. Sterile seedlings were cultured using seeds of "CCRI 24", and hypocotyls were taken and cut off for callus cultivation on the callus induction medium (without supplements for the control; supplemented with DIECA (also known as a copper reagent, Sangon Biotech, Cat. No.: A600846) at final concentrations of 200 µM, 400 µM and 800 µM; or supplemented with ibuprofen (also known as Ibuprofen, Cat. No.: HY-78131) at final concentrations of 20 µM, 40 µM and 80 µM) after 7 days. Explants from "CCRI 24" were cut to consistent length and placed under the same conditions for cultivation. After one month, the growth of callus was observed and photographed with a camera and microscope. Statistics on the weights of callus and measurement of JA content were performed. Samples were taken for fluorescence quantitative PCR to detect the expression of cell cycle-related genes (the steps of method and primers were the same as Example 1).

### Method for determining JA content:

1) Method for extracting jasmonic acid: About 0.1 g of samples was weighed, with addition of 1 mL of pre-cooled reagent I (80% acetonitrile) for extraction overnight at 4°C, followed by centrifugation at 8,000 g for 10 min. The residue obtained was extracted with 0.5 mL of reagent I (80% acetonitrile) for 2 hours. Then centrifugation was perfomed at 8,000 g for 10 min again, and the supernatants obtained from the two centrifugations were pooled for drying by blowing with nitrogen at 40°C, followed by addition of 0.5 mL of water for reconstitution, and then 0.5 mL of reagent II (petroleum ether) was added for extraction and decolorization at 60°C-90°C for three times, with the upper ether phase discarded. Reagent III (saturated citric acid aqueous solution) was added and adjusted to pH 2.5-3.0, followed by extraction with equal volume of reagent IV (ethyl acetate) for three times after mixing well, with the organic phase pooled, which was blown with nitrogen to dry. 0.2 mL of reagent V (ether:methanol = 9:1, volume ratio) was added for reconstitution, and then 20 µL of reagent VI (2 mmol/L trimethylsilanized diazomethane (dissolved in n-hexane) were added and placed at 25°C for 30 min after mixing well. 20 µL of reagent VII (2 mol/L glacial acetic acid in n-hexane) were added and placed at 25°C for 30 min after mixing well, which was blown with nitrogen to dry in the ice water bath. 0.5 mL of methanol was added and dissolved by vortex shaking, followed by filtration through a needle filter and ready for testing.
2) HPLC conditions: Chromatograph: RIGOL L3000 High Performance Liquid Chromatograph, UV wavelength of 210 nm.
Column: RIGOL C18 reverse phase column (250 mm * 4.6 mm, 5 µm)

Column temperature: 35°C, flow rate: 0.8 mL/min, injection volume: 10 µL. Preparation of the mobile phase: Mobile phase A: acetonitrile, B: 0.1% phosphoric acid aqueous solution, isocratic elution, (60% of A + 40% of B, % represents volume percent).

The results are shown in FIG. 2, both DIECA and ibuprofen could significantly inhibit the growth of callus of cotton and the JA content as also significantly decreased with marked changes in cell cycle-related genes of callus, indicating that both JA synthesis inhibitors can inhibit the growth of callus of cotton.

### Example 3. Phylogenetic analysis and functional study of JAZ gene in plants

### 1. Distribution and phylogenetic analysis of JAZ and MYC genes in various organisms

JAZ protein in the biosphere was analyzed for gene homology using MEGA 7 software, and plotted into a phylogenetic tree. As shown in A of FIG. 3, the analysis results showed that JAZ was only present in autotrophic organisms (such as 9 in *Physcomitrella patens* in Bryophyta phylum, 1 in *Marchantia potymorpha* in Bryophyta, 2 in *Cycas micholitzii* in gymnosperm, 7 in *Spirodela polyrrhiza* in monocotyledonous, and 35 in the cotton in dicotyledonous), while absent in both autotrophic organisms (such as *Chlamydomonas reinhardtii*) and heterotrophic organisms (such as viruses, bacteria, fungi, lower animals, higher animals, and humans). A total of 35 JAZ members (amino acid sequences as set forth in SEQ ID No.1 to SEQ ID No.35, and the corresponding encoding genes in order are as set forth in SEQ ID No.36 to SEQ ID No.70) were identified in upland cotton, which can be mainly divided into 6 major categories represented by GhJAZ1, GhJAZ7, GhJAZ9, GhJAZ10, GhJAZ11, and GhJAZ31 based on the phylogenetic analysis (B in FIG. 3).

MYC belongs to the widely existing bHLH transcription factor family in the biosphere, and in terms of evolutionary relationships, MYC is present in viruses, prokaryotes, and eukaryotes (A in FIG. 4). MYC2 had been most extensively studied in plants as a regulatory hub within the JA signaling pathway. The carboxyl domain of MYC2 contains a conserved bHLH domain, which is capable of forming homodimers or heterodimers with other proteins and binding to the G-box (5'-CACGTG-3') in the promoters of gene of interest. MYC2 contains a transcriptional activation domain (TAD) at its amino terminus, through which it recruits the desired mediator complex required for transcriptional initiation that specifically interacts with the plant mediator complex MED25. From the results of amino acid homology alignment, MYC is more conserved in TAD and bHLH domains (B and C in FIG. 4).

### 2. A study on proteins and protein complexes interacting with JAZ gene in cotton

The study on Arabidopsis had shown that the inhibition of JAZ protein on JA signaling was mainly achieved by inhibiting the transcription factor function of MYC, which had been experimentally verified by us in cotton. Based on the phylogenetic tree of Arabidopsis and cotton JAZs, we screened 6 cotton JAZs (GhJAZ1, GhJAZ7, GhJAZ9, GhJAZ10, GhJAZ11 and GhJAZ31), which were analyzed with GhMYC2, GhNINJA, GhTPL, GhHDA6, and GhCOI1 for yeast two hybrid. The amino acid sequence of GhJAZ1 was set forth in SEQ ID No.4, and the corresponding encoding gene sequence was set forth in SEQ ID No.39; the amino acid sequence of GhJAZ7 was set forth in SEQ ID No.7, and the corresponding encoding gene sequence was set forth in SEQ ID No.42; the amino acid sequence of GhJAZ9 was set forth in SEQ ID No.2, and the corresponding encoding gene sequence was set forth in SEQ ID No.37; the amino acid sequence of GhJAZ10 was set forth in SEQ ID No.3, and the corresponding encoding gene sequence was set forth in SEQ ID No.38; the amino acid sequence of GhJAZ11 was set forth in SEQ ID No.5, and the corresponding encoding gene sequence was set forth in SEQ ID No.40; and the amino acid sequence of GhJAZ31 was set forth in SEQ ID No.9, and the corresponding encoding gene sequence was set forth in SEQ ID No.44. The amino acid sequence of GhMYC2 was set forth in SEQ ID No.71, and the corresponding encoding gene sequence was set forth in SEQ ID No.72; the amino acid sequence of GhNINJA was set forth in SEQ ID No.73, and the corresponding encoding gene sequence was set forth in SEQ ID No.74; the amino acid sequence of GhTPL was set forth in SEQ ID No.75, and the corresponding encoding gene sequence was set forth in SEQ ID No.76; the amino acid sequence of GhHDA6 was set forth in SEQ ID No.77, and the corresponding encoding gene sequence was set forth in SEQ ID No.78; and the amino acid sequence of GhCOI1 was set forth in SEQ ID No.79, and the corresponding encoding gene sequence was set forth in SEQ ID No.80.

Method of yeast two hybrid experiment: Cotton JAZ yeast plasmids such as PGBKT7::GhJAZ1(DNA fragment set forth in SEQ ID No.39 inserted between BamHl and EcoRl of pGBKT7 plasmid), PGBKT7::GhJAZ9(DNA fragment set forth in SEQ ID No.37 inserted between BamHl and EcoRl of pGBKT7 plasmid), PGBKT7::GhJAZ7(DNA fragment set forth in SEQ ID No.42 inserted between BamHl and EcoRl of pGBKT7 plasmid), PGBKT7::GhJAZ10(DNA fragment set forth in SEQ ID No.38 inserted between BamHl and EcoRl of pGBKT7 plasmid), PGBKT7::GhJAZ11(DNA fragment set forth in SEQ ID No.40 inserted between BamHl and EcoRl of pGBKT7 plasmid), PGBKT7::GhJAZ31(DNA fragment set forth in SEQ ID No.44 inserted between BamHl and EcoRl of pGBKT7 plasmid), PGBKT7::GhJAZ1^{ΔNT} (the sequence of gene GhJAZ1^{ΔNT} set forth in SEQ ID No.84 inserted between BamHl and EcoRl of pGBKT7 plasmid and GhJAZ1^{ΔNT} protein sequence set forth in SEQ ID No.83), PGBKT7::GhJAZ1^{ΔZIM} (the sequence of gene GhJAZ1^{ΔZIM} set forth in SEQ ID No.86 inserted between BamHl and EcoRl of pGBKT7 plasmid and GhJAZ1^{ΔZIM} protein sequence set forth in SEQ ID No.85), and PGBKT7::GhJAZ1^{ΔJas} (the sequence of gene GhJAZ1^{ΔJas} set forth in SEQ ID No.88 inserted between BamHl and EcoRl of pGBKT7 plasmid and GhJAZ1^{ΔJas} protein sequence set forth in SEQ ID No.87), and vectors plasmids such as PGADT7::GhMYC2(DNA fragment set forth in SEQ ID No.72 inserted between BamHl and EcoRl of pGADT7 plasmid), PGADT7::GhNINJA (DNA fragment set forth in SEQ ID No.74 inserted between BamHl and EcoRl of pGADT7 plasmid), PGADT7::GHTPL ( DNA fragment set forth in SEQ ID No.76 inserted between BamHl and EcoRl of pGADT7 plasmid), PGADT7::GhHDA6(DNA fragment set forth in SEQ ID No.78 inserted between BamHl and EcoRl of pGADT7 plasmid), and PGADT7::GhCOI1(DNA fragment set forth in SEQ ID No.80 inserted between BamHl and EcoRl of pGADT7 plasmid), were constructed using BamHl and EcoRl restriction endonucleases, followed by separate co-transfection (one was pGBKT7 + gene plasmid and the other was pGADT7 + gene plasmid) into the yeast competent cells Y2HGold (Shanghai Weidi Company, Cat. No.: YC1002) after sequencing verification, and then Carrier DNA (95°C, 5 min) and PEG/LiAC were added separately and mixed well, followed by being placed successively in the water bath at 30°C for 30 min (flipped 6-8 times at 15 min and mixed well) and at 42°C for 15 min (flipped 6-8 times at 7.5 min and mixed well), and centrifuged at 5,000 rpm for 40 s with the supernatant discarded, and then ddH₂O was added for resuspending, followed by centrifugation at 5,000 rpm for 30 s with the supernatant discarded, and addition of ddH₂O again for resuspending, which was plated on the two-deficient medium (SD/- Leu/-Trp) for cultivation at 28°C for 1-2 days. The colonies were picked after growing out and placed on the four-deficient medium (SD-Ade/-His/-Leu/-Trp, supplemented with Aba and X-α-gal) for cultivation at 28°C for 3-4 days, followed by observation of the interaction.

Method of the luciferase experiment: GhJAZ1(corresponding encoding gene sequence set forth in SEQ ID No.39) was subcloned into pCAMBIA-CLuc vector (Chen, H., et al. "Firefly Luciferase Complementation Imaging Assay for Protein-Protein Interactions in Plants." Plant Physiology 146.2(2008):368-376.) between KpnI and SalI cleavage sites, and the transcription factors such as GhMYC2(corresponding encoding gene sequence set forth in SEQ ID No.72), GhNINJA (corresponding encoding gene sequence set forth in SEQ ID No.74), and GhCOI1(corresponding encoding gene sequence set forth in SEQ ID No.80) were subcloned into pCAMBIA-NLuc (Chen, H., et al. "Firefly Luciferase Complementation Imaging Assay for Protein-Protein Interactions in Plants." Plant Physiology 146.2(2008):368-376.) between KpnI and SalI cleavage sites, respectively. The constructed plasmids were then transformed into Agrobacterium GV3101(pSoup-p19) (Shanghai Weidi Company, Cat. No.: AC1003), and placed successively on ice for 5 min, in liquid nitrogen for 5 min, in water bath at 37°C for 5 min, and on ice for 5 min, and then LB liquid medium was added for cultivation at 28°C and 220 rpm for 2-3h, followed by plating onto LB solid plates containing corresponding antibiotics (kanamycin + rifampicin) for cultivation at 28°C for 2-3 days. Positive monoclonal clones were picked for cultivation after colonies grown out. The corresponding NLUC and CLUC vectors then were mixed and injected into tobacco leaves (1 day in the dark/1 day in the light). Luciferase substrates were applied at the infected leaves before taking images and placed in a dark room for at least 6 min to quench fluorescence, followed by observation on the Nightowl-II LB983 instrument, using IndigoBasic software under the luciferase-5min setting conditions.

Steps of method of CO-IP experiment: Non-denaturing lysate was added to 293T cell culture plate (Pricella, Cat. No.: CL-0005), and the cells were completely lysed at 4°C. The lysate was centrifuged at 12,000 rpm for 10 min and the supernatant was collected. The corresponding antibody was added to the obtained non-denatured protein solution, which was incubated overnight at 4°C. Pierce^{™} protein A/G magnetic agarose (Thermo Fisher Scientific, Inc.) were washed with lysis buffer, followed by addition pre-treated beads to the cell lysate, and incubation at room temperature for 2 hours. The mixture was centrifuged at 4°C, 2,500 rpm for 5 min with supernatant removed, and the agarose beads were washed 5 times with 1ml of lysis buffer. An appropriate amount of protein loading buffer was added to the pellet, and the sample was incubated in water bath at 100°C for 10 min. The band of interest was detected by Western blotting.

The results of yeast two hybrid showed that the interaction of GhJAZ1 with GhMYC2 was strong, and the interaction of GhJAZ9 with GhMYC2 was weak (A in FIG. 5). The results of the luciferase experiment showed that stronger fluorescence was observed on the tobacco that was injected with a combination of GhJAZ1 Agrobacterium and GhMYC2 Agrobacterium after applying the luciferase substrate, while no fluorescence was observed on the control (B in FIG. 5); and weaker fluorescence was observed on the tobacco that was injected with a combination of GhJAZ9 Agrobacterium and GhMYC2 Agrobacterium after applying the luciferase substrate, while no fluorescence was observed on the control (C in FIG. 5); which further demonstrating that there was a strong interaction of GhJAZ1 with GhMYC2, while a weak interaction of GhJAZ9 with GhMYC2.

The interaction of GhJAZ1 with the major proteins in the transcriptional inhibition complex of the JA signaling pathway was explored by yeast two hybrid, the results from which showed that GhJAZ1 interacted with GhNINJA, GhTPL, and GhHDA6(D in FIG. 5), and GhJAZ1 interacted with GhCOI1 in the presence of JA He (D in FIG. 5). The results of the luciferase experiment showed that stronger fluorescence was observed on the tobacco that was injected with a combination of GhJAZ1 Agrobacterium and GhNINJA Agrobacterium after applying the luciferase substrate, while no fluorescence was observed on the control (E in FIG. 5); and weaker fluorescence was observed on the tobacco that was injected with a combination of GhJAZ1 Agrobacterium and GhCOI1 Agrobacterium after applying the luciferase substrate, while no fluorescence was observed on the control (E in FIG. 5), demonstrating the interaction between GhJAZ1 and GhNINJA and GhCOI1. The CO-IP results showed that GhJAZ1 interacts with GhTPL and GhHDA6(G and H in FIG. 5). The above results indicated that a transcriptional inhibition complex was formed by JAZ1 with MYC2, NINJA, TPL and the like, and thus inhibited the transcriptional activity of MYC2.

Exploration of functional domains for the interaction was critical for the development of functional polypeptides. The interaction of GhJAZ1 after deletion of functional domains with GhMYC2, GhNINJA, GhTPL, GhHDA6, and GhCOI1 was investigated by yeast two hybrid. The results showed that the interaction of GhJAZ1 after deletion of NT and ZIM domain with GhMYC2 was significantly weakened, the interaction of GhJAZ1 after deletion of ZIM domain with GhNINJA was significantly weakened, the interaction GhJAZ1 after deletion of NT and Jas domain deficient GhJAZ1 with GhTPL was significantly weakened, the interaction of GhJAZ1 after deletion of NT domain with GhHDA6 was significantly weakened, and the interaction of GhJAZ1 after deletion of NT, ZIM and Jas domain with GhCOI1 was significantly weakened, indicating that NT in GhJAZ1 mainly involved in the interaction with GhMYC2, GhTPL, GhHDA6, and GhCOI1, ZIM domain involved in the interaction with GhMYC2, GhNINJA, and GhCOI1, and Jas domain involved in the interaction with GhTPL and GhCOI1(D in FIG. 5).

### Example 4. Analysis of anti-tumor effect of cotton JAZ protein

### 1. Analysis of similarities and differences between plant tumors and animal tumors

There were many similarities between plant callus (plant tumors) and human tumors, such as similar morphology; induced by endogenous and exogenous stimuli; long-lasting regenerative and self-replicating abilities; rapid cell division, rapid self-proliferation, and unstable cell growth; and both were regulated by transcription factors such as MYC. The growth of "plant tumor" cannot develop to be malignant due to the strict regulation by JA signaling, whereas human tumor often deteriorates into cancer due to its uncontrollability, which is different from the "plant tumor" that is controllable. Cells from plant callus had totipotency and can differentiate into a variety of other tissue cells; but cells formed by cancer cell differentiation were still cancer cells, and they also had the characteristics of loss of contact inhibition, weakened adhesion between cancer cells, and decreased adhesiveness (A in FIG. 6).

### 2. Screening and validation of the interaction of cotton JAZ protein with human MYC protein

The encoding genes of the six JAZ representatives in cotton (GhJAZ1, GhJAZ7, GhJAZ9, GhJAZ10, GhJAZ11, GhJAZ31, see Example 3 for specific related sequences) were constructed into the pGBKT7 vector (YouBio, Cat. No.: VT1638) between EcoRI and BamHI, respectively, and simultaneously, the MYC oncogene (c-Myc (NCBI accession number: NM_002467.6), NMYC (NCBI accession number: NP_005369.2) and LMYC (NCBI accession number: NP_001028254.2) were constructed into the pGADT7 vector (YouBio, Cat. No.: VT1639) between EcoRI and BamHI for the interaction analysis by yeast two hybrid, respectively (the method as Example 3). The results showed that GhJAZ1 interacted with both c-Myc and NMYC simultaneously (B in FIG. 6).

Simultaneously, the interaction was validated by GST Pull down experiment. Firstly, pET-30a-GhJAZ1(the DNA sequence set forth in SEQ ID No.39 was inserted into pET-30a (+) vector (YouBio, Cat. No.: VT1212) between NdeI and HindIII) and pGEX-4T-1-c-Myc vectors (the CDS sequence of c-Myc (NCBI accession number: NM_002467.6) was inserted into pGEX-4T-1 vector (YouBio, Cat. No.: VT1253) between BamHI and EcoRI) was constructed and transformed into *Escherichia coli* BL21, which was placed on ice for 30 min, followed by heat shock at 42°C for 60 s, and then placed on ice for 2 min, and then LB medium was added for cultivation at 37°C and 200 rpm for 1 h, which was plated onto LB plates containing corresponding antibiotics for cultivation at 37°C. After colonies grown out, monoclonal clones were picked and shaking for incubation and expanded until the medium began to turn slightly white, and then different concentrations of IPTG were added for induction and cultured overnight under different temperatures setting. Subsequently, centrifugation at 5,000 rpm, 4°C was carried out for 10 min, with addition of an equal volume of PBS (supplemented with PMSF and DTT) for resuspending. It was crushed with ultrasonic waves for 5 s, then put on ice for 5 s, until the resuspended solution became obviously clear. Centrifugation at 13,000 rpm, 4°C was carried out for 10 min to separate the supernatant and inclusion bodies. The supernatant was transferred into a protein purification column, and washed and eluted following incubation, and then proteins were detected and collected dropwise using protein pre-staining solution. After the above expression and purification, GST-c-Myc and His-GhJAZ1 fusion proteins were obtained. Then GST beads were washed, and 2 µg of GST-c-Myc fusion protein was added and incubated for 2 h, subsequently, washed again, and 1 µg of His GhJAZ1 fusion protein was added, and from which a portion was pipetted as Input and incubated for 1 h, finally, it was washed, followed by addition of the loading buffer for WB test. The results also demonstrated that GhJAZ1 interacted with c-Myc (C in FIG. 6).

Immunoprecipitation method (method of the experiment as Example 3) was used. The CO-IP results showed that GhJAZ1 interacted with c-Myc and NMYC in HEK293 cells (D and E in FIG. 6).

Using the method of LUC experiment, c-Myc (NCBI accession number: NM_002467.6) was subcloned to pCAMBIA NLuc vector (Chen, H., et al. "Firefly Luciferase Complementation Imaging Assay for Protein-Protein Interactions in Plants." Plant Physiology 146.2(2008):368-376.) between the KpnI and SalI cleavage sites, and GhJAZ1(SEQ ID No.39) was subcloned to pCAMBIA-CLuc vector (Chen, H., et al. "Firefly Luciferase Complementation Imaging Assay for Protein-Protein Interactions in Plants." Plant Physiology 146.2(2008):368-376.) between the KpnI and SalI cleavage sites. The constructed recombinant plasmids verified to be positive by sequencing were transformed into Agrobacterium GV3101(pSoup-p19) (Shanghai Weidi biology technology co., Ltd, Cat. No.: AC1003), which were co-transfected into tobacco leaves. 2-3 days after injection, the infected leaves coated with the fluorescent substrate were placed in a dark chamber of the instrument for at least 6 min before taking images to quench fluorescence, followed by observation on the Nightowl-II LB983 instrument, using IndigoBasic software under the luciferase-5min setting conditions. Cultivation was performed after co-infection. If there is interaction, LUC luciferase is expressed, and luminescence is detected after substrate application. As shown in F in FIG. 6, GhJAZ1 interacts with c-Myc.

GhJAZ1(SEQ ID No.39) was subcloned into pUC-SPYNE vector (Song, Yun, et al. "BIN2 negatively regulates plant defense against Verticillium dahliae in Arabidopsis and cotton." Plant Biotechnology Journal (2021)) between the BamHI and XbaiI cleavage sites by the bimolecular fluorescence complementary experiment to construct pUC-SPYNE-GhJAZ1. c-Myc (NCBI accession number: NM_002467.6) was subcloned into pUC-SPYCE vector (Song, Yun, et al. "BIN2 negatively regulates plant defense against Verticillium dahliae in Arabidopsis and cotton." Plant Biotechnology Journal (2021)) between BamHI and XbaiI cleavage sites to construct pUC-SPYCE-c-Myc. Tobacco leaf tissues were immersed transiently in Agrobacterium GV3101 which was co-transformed with PUC-SPYNE-GhJAZ1 and pUC-SPYCE-c-Myc, followed by observation of YFP signals under a laser confocal microscope, as shown in FIG. 6, indicating the interaction of GhJAZ1 with c-Myc in the nucleus of tobacco.

### 3. Testing of inhibitory effect of GhJAZ1 protein on tumor cells

CCK8 cell viability detection: cell suspension was seeded in 96-well plates at a concentration of 5 × 10⁴ cells/ml (100 µL/well). The plates were placed in the incubator for pre-culture (at 37°C, 5% CO₂), and control and protein agents were added to each well 24 h after seeding. The plates were then placed in the incubator for 24 h, followed by addition of 10 µL CCK8 solution into each well and incubation of the plates in the incubator for 0.5-2 h before the absorbance measurement at 450 nm using a microplate reader.

GhJAZ1 had a strong interaction with the proto-oncogenes c-Myc and NMYC. We constructed GhJAZ1 prokaryotic expression plasmid with His-tag to express proteins (see step 2 in Example 4), and then a GhJAZ1 protein, along with 6 His tags, which was approximately 30 KD in size was found by WB detection (A in FIG. 7). The purified GhJAZ1 protein was used to treat breast cancer cells (MCF7) and breast epithelial cells (MCF10A), and the cell viability was measured with CCK8. The results showed that with the increase of GhJAZ1 protein concentration, the viability of MCF7 cells decreased significantly (B in FIG. 7), while the viability of MCF10A cells did not change significantly (C in FIG. 7).

Method for apoptosis staining. Fluorescence microscopy detection: a. Seeding and culturing. Cells were seeded onto multiwell plates such as 96-well plates, cell culture dishes, or cell slides, and were treated in a certain way according to the experimental design. b. Washing (optionally). For adherent cells, the medium was removed, and cells were washed once with PBS. For suspended cells, cells were centrifuged at 250-1000× g at room temperature for 5 min, with the supernatant removed, followed by washing once with PBS. Phenol red or serum might interfere with the detection of this kit, therefore, a vacuum pump was preferred when removing the medium and PBS. PBS washing was not necessary in the case that the residual liquid can be sufficiently aspirated. c. Staining. An appropriate volume of Calcein AM/PI detection working solution (Beyotime Biotech. Inc., Calcein/PI Cell Viability and Cytotoxicity Detection Kit, Cat. No.: C2015) was added. Usually, 100 µl were added to each well for a 96-well plate, 250 µl for a 24-well plate, 500 µl for a 12-well plate, and 1 ml for a 6-well plate. Incubation was performed at 37°C in dark for 30 min. The optimal incubation time varies for different cells, with 30 min as the initial incubation time. Subsequently, the staining time might be adjusted and optimized according to the actual staining effect to obtain a more ideal staining effect. d. Detection. After incubation, the staining effect was observed under a fluorescence microscope (with Calcein AM in green fluorescence, Ex/Em = 494/517 nm; PI in red fluorescence, Ex/Em = 535/617 nm). It was noted that the whole procedures should be carried out in dark.

Apoptosis staining was performed on cells, and the results showed that the mortality rate of MCF7 cells significantly increased after treatment with GhJAZ1 protein (D-K in FIG. 7). We further treated lung cancer cells A549, ovarian cancer cells SKOV3, and cervical cancer cells Hela with GhJAZ1 protein, and found that these cancer cells were all inhibited by GhJAZ1 protein (L-N in FIG. 7). These experimental results indicated that GhJAZ1 protein can efficiently and broadly inhibit cancer cells.

### 4. Analysis of effect of GhJAZ1 protein on the cell cycle and apoptosis of tumor cells

In order to further explore the molecular mechanism of inhibition of tumor cells by GhJAZ1 protein, the apoptosis and cell cycle of MCF7 breast cancer cells treated with GhJAZ1 (using 0.691 µM of IC50 concentration) protein and normal breast epithelial cells MCF10A were detected after 24 h.

Apoptosis detection: cells were collected in 1.5 EP tubes and centrifuged at 300 g for 5 min, with the supernatant discarded, followed by collecting the cells, washing with PBS and resuspending. 1 × 10⁵ resuspended cells were taken and centrifuged at 300 g for 5 min, with the supernatant discarded. The cells were washed with PBS, resuspended by adding 500 µl of diluted 1 × Annexin V Binding Buffer working solution, and then a staining solution of 5 µL of Annexin V-FITC and 5 µL of propidium iodide (PI) were added and mixed well by vortex, followed by incubation at room temperature in the dark for 15-20 min, and detection on the instrument. Specifically, the Annexin V-FITC/PI fluorescent dual staining cell apoptosis detection kit (Wuhan Pricella Life Science&Technology Co.,Ltd., Cat. No.: P-CA-201) was used.

Cell cycle detection: cells were moderately digested by trypsin, and collected by centrifugation with supernatant removed, and then cells were washed with PBS, and collected by centrifugation at 1,500 rpm for 5 minutes to prepare a single-cell suspension (at a concentrationof 1 × 10⁶/ml). 1 ml of single-cell suspension was then taken and centrifuged with the supernatant discarded, and then 500 µl of 70% pre-cooled ethanol were added to fix the cells overnight at 4°C. The next day, cells were collected by centrifugation, resuspended with 1 ml of PBS and centrifuged, and then 100 µl of RNase A solution were added to the cell pellet, followed by placement in water bath at 37°C for 30 min, and then 400 µl of PI staining solution was added and mixed well, followed by incubation at 4°C in dark for 30 min and detection on the instrument to record under red fluorescence at the excitation wavelength of 488 nm. Specifically, a DNA content detection kit (cell cycle) (Solarbio, Cat. No.: CA1510) was used.

The results of cell apoptosis detection showed that GhJAZ1 led to an increased apoptosis rate of cancer cells (A-C in FIG. 8), while had no significant effect on normal cells (D-F in FIG. 8). The cell cycle detection results showed that GhJAZ1 treatment resulted in a decrease in the proportion of S-phase cells, an increase in the proportion of G2-M phase cells, and cell cycle block in G2-M phase (G-I in FIG. 8), while no G2-M phase block shown in MCF10A cells (J-L in FIG. 8).

### 5. Regulation of downstream genes of c-Myc by GhJAZ1

In order to further explore the mechanism of inhibition of cancer cell proliferation by GhJAZ1, we performed transcriptome sequencing on MCF7 cells treated with GhJAZ1 protein (using 0.691 µM of IC50 concentration) for 24 h, and the results showed that the expression of cell cycle-related genes was down regulated (A in FIG. 9). And it was validated to be consistent with the transcriptome results (B in FIG. 9) by RT-PCR (the steps of method as Example 1). Wherein, the sequences of RT-PCR primers were as follows:
Actin was used as the internal reference and the detection primers were:
   Human-actin-F: 5-CATGTACGTTGCTATCCAGGC-3;
   Human-actin-R: 5-CTCCTTAATGTCACGCACGAT-3.
Primers for detecting DHFR were:
   Q-DHFR-F: 5-ATGGTTGGTTCGCTAAACTGCATC-3;
   Q-DHFR-R: 5-CCTTGAGTTCTCTGCTGAGAACTAAATTAA-3.
Primers for detecting CDC45 were:
   Q-CDC45-F: 5-CTTTGACTACGAGCAGTATGAATATCATG-3;
   Q-CDC45-R: 5-TGTGCAGTCCACGGAGAGTGTG-3.
Primers for detecting CDCA7 were:
   Q-CDCA7-F: 5-CTGATGACAGTTGTGACAGCTTTGC-3;
   Q-CDCA7-R: 5-GAATCGGAGTTGGAATCAGTCACA-3.
Primers for detecting CDC 16 were:
   Q-CDC16-F: 5-ATTGTATGAAGCATGTCGTTACCTTGC-3;
   Q-CDC16-R: 5-TGTAGGTAGCCAGGGTTCGGTTAT-3.
Primers for detecting POLD1 were:
   Q-POLD1-F: 5-AGGATGCCTACCTGCCACTG-3;
   Q-POLD1-R: 5-GGTACAGCGAGGAGAAGTCCAGG-3.
Primers for detecting POLK were:
   Q-POLK-F: 5-AGACAAGCTGTGATGGACTTCATCAA-3;
   Q-POLK-R: 5-CTCTTCCGCTTTATTTATCTCACTGAATG-3.

According to the downstream genes of c-Myc reported in literatures, further validation was conducted by RT-PCR, and the results showed that in the cancer cell MCF7 treated with GhJAZ1, the apoptosis related genes Bcl2, BAX, HK2, FASN, and MCM5 downstream of c-Myc were extremely significantly downregulated, while the P21 gene was extremely significantly upregulated; the cell cycle-related genes CCNA2, CCNB1, and CCND1 were extremely significantly downregulated, while CCNE1 was extremely significantly upregulated (C in FIG. 9). Whereas in the normal cell MCF10A, only Bcl2 and MCM5 were significantly decreased but at a minor extent, while P21C, CNB1, CCND1, and CCNE1 were all significantly increased, but also at a minor extent, and there was no significant trend overall (D in FIG. 9). Wherein, the sequences of primers were as follows:
Actin was used as the internal reference and the detection primers were:
   Human-actin-F: 5-CATGTACGTTGCTATCCAGGC-3;
   Human-actin-R: 5-CTCCTTAATGTCACGCACGAT-3.
Primers for detecting c-MYC were:
   Q-c-MYC-F: 5-ATGGTGACCGAGCTGCTGGG-3;
   Q-c-MYC-R: 5-CCACCGAGGGGTCGATGCA-3.
Primers for detecting P21 were:
   Q-P21-F: 5-GATGTCCGTCAGAACCCATGCG-3;
   Q-P21-R: 5-CGCTCCCAGGCGAAGTCACC-3.
Primers for detecting Bcl2 were:
   Q-Bcl2-F: 5-GGGTACGATAACCGGGAGATAGTGATG-3;
   Q-Bcl2-R: 5-GCACCGGGCTGAGCGCAG-3.
Primers for detecting BAX were:
   Q- BAX-F: 5-GAGGCACCCGAGCTGGCC-3;
   Q- BAX-R: 5-TGATCAGTTCCGGCACCTTGGT-3.
Primers for detecting HK2 were:
   Q-HK2-F: 5-CGCTGTGGTGGACAGGATACGAG-3;
   Q-HK2-R: 5-CTATCGCTGTCCAGCCTCACGGA-3.
Primers for detecting FASN were:
   Q-FASN-F: 5-GGAAGCTGCCAGAGTCGGAGAACTT-3;
   Q-FASN-R: 5-GTCCACGATGGCTTCATAGGTGACTT-3.
Primers for detecting MCM5 were:
   Q-MCM5-F: 5-ATGTCGGGATTCGACGATCCTG-3;
   Q-MCM5-R: 5-TGTAATGCCGCTTGAGTTCATCCC-3.
Primers for detecting CCNA2 were:
   Q-CCNA2-F: 5-CTATCCTCGTGGACTGGTTAGTTG-3;
   Q-CCNA2-R: 5-AACTTTGAGGCTAACAGCATAGCA-3.
Primers for detecting CCNB1 were:
   Q-CCNB1-F: 5-GAAATTCAGGTTGTTGCAGGAGAC-3;
   Q-CCNB1-R: 5-GTGCTTAGTATAAGTGTTGTCAGTCACAAA-3.
Primers for detecting CCND1 were:
   Q-CCND1-F: 5-AGCTCCTGTGCTGCGAAGTGGA-3;
   Q-CCND1-R: 5-GACAGGAAGCGGTCCAGGTAGTTCA-3.
Primers for detecting CCNE1 were:
   Q-CCNE1-F: 5-TTTACCCAAACTCAACGTGCAAG-3;
   Q-CCNE1-R: 5-TAGACTTCACACACCTCCATTAACCA-3.

### Example 5. Analysis of GhJAZ1 protein entry into cancer cells and cell nuclei

### 1. Screening and validation of the interaction of GhJAZ1 with surface receptor proteins for the tumor cells

In order to explore how GhJAZ1 enters cancer cells, we cloned the genes of most tumor surface receptors, including uPAR (NCBI database accession number of NM_002659.4), CCK1R (NP_000721.1), CCK2R (NP_001350481.1), HER2(NP_004439.2), GRPR (NP_005305.1), MUC1(NP_001358649.1), VEGFR1 (NP_002010.2), Insulin receptor (NM_000208.4), NTR (XM_011528827.2), PSMA (XM_017017432.1), TFR1(NM_001128148.3), TFR2(NM_003227.4), PEPT1 (NM_005073.4), APN (NM_001150.3). The receptor proteins that interact with GhJAZ1 were then screened by yeast two hybrid (the steps of the experiment as Example 3). The results showed that GhJAZ1 interacted with uPAR (A in FIG. 10). The CO-IP experiment was further conducted (the steps of the method as Example 3), and the results showed that GhJAZ1 interacts with uPAR *in vivo* (B in FIG. 10). We detected the interaction of GhJAZ1 with uPA (as the ligand of uPAR, NCBI database accession number of NP_002649.2) and uPAR by yeast two hybrid (the steps of the experiment as Example 3). The results showed that GhJAZ1 interacts with the ATF domain of uPA, and the ATF domain of uPA also interacts with uPAR (C and D in FIG. 10).

### 2. Detection of GhJAZ1 protein entry into the nucleus

GhJAZ1 enters cells through the cancer cell surface receptor uPAR, there is a question regarding if it could enter into the nucleus. We further designed and constructed a prokaryotic expression vector. GhJAZ1+RFP (the amino acid sequence set forth in SEQ ID No.81, corresponding encoding gene sequence set forth in SEQ ID No.82) was ligated to pET-30a (+) vector (YouBio, Cat. No.: VT1212) between NdeI and HindIII, and GhJAZ1 + RFP protein with red fluorescence was prokaryotically expressed and purified (the step of method as step 2 in Example 4) (A in FIG. 11), and then MCF7 cells were treated with GhJAZ1 + RFP protein and observed by a fluorescence microscope. The results showed no red fluorescence was observed in the control (B-E in FIG. 11), red fluorescence was observed outside the cell nucleus with the addition of IPR803(uPAR inhibitor at a concentration of 50 µM) (MedChemExpress company, Cat. No.: HY-111192) (F-I in FIG. 11), and fluorescence distributed in a dotted pattern and merged with the blue fluorescence from the nucleus (J-M in FIG. 11) with addition of GhJAZ1 + RFP protein (at a concentration of 0.5 µM), demonstrating that GhJAZ1 entered the nucleus.

### Example 6. Analysis of anti-tumor effect of GhJAZ1 on ovarian cancer mouse model in vivo

Human ovarian cancer cells SKOV3 were cultured. Cells were digested by the trypsin before being used for tumor bearing. Cells were washed twice with PBS, followed by resuspending with PBS to a concentration of 1 × 10⁸/mL. The cell suspension was subcutaneously injected into the axilla of nude mice, with an inoculation dose of 0.2 mL per nude mouse. After inoculation, nude mice were randomly assigned into two groups, which were PBS group and GhJAZ1 protein (5 mg/kg) treated group, respectively, with 8 mice in each group. After tumor inoculation, administration began when the tumor volume reached to about 50-100 mm³. Administration by intratumoral injection was conducted every 2 days for a cycle of 14 days, in total of 7 times. Throughout the entire experiment, the body weight of nude mice was monitored while administration, and tumor size was measured using a vernier caliper according on the formula V = 1/2 × length × width × width, to calculate the tumor volume, and plot tumor growth curve. 14 days later, the tumor was removed, took images and weighted.

The results of intratumoral injection showed that as the injection days progressed, the volume of tumors in the mice injected with GhJAZ1 protein was highly significantly lower than that in the control group (B in FIG. 12). A and C in FIG. 12 showed the mice and the tumors removed 14 days after injection, and the weight of tumors in the injection group was highly significantly lower than that in the control group (D in FIG. 12).

### Example 7. Analysis of the domain of GhJAZ1 interacted with uPAR and c-Myc by yeast two hybrid

Cotton JAZ yeast plasmids such as PGBKT7::GhJAZ1, PGBKT7::GhJAZ1^{ΔNT}, PGBKT7::GhJAZ1^{ΔZIM} and PGBKT7::GhJAZ1^{ΔJaS}(see Example 3), and vector plasmids such as PGADT7::uPAR (uPAR with NCBI database accession number of NM_002659.4), PGADT7::uPAR-D1D2(the encoding gene sequence of uPAR-D1D2 set forth in SEQ ID No.90, and the corresponding amino acid sequence set forth in SEQ ID No.89), PGADT7::uPAR-D1HD3(the encoding gene sequence of uPAR-D1HD3 set forth in SEQ ID No.92, and the corresponding amino acid sequence set forth in SEQ ID No.91), PGADT7::uPAR-D2D3(the encoding gene sequence of uPAR-D2D3 set forth in SEQ ID No.94, and the corresponding amino acid sequence set forth in SEQ ID No.93), PGADT7::c-Myc (NCBI accession number of c-Myc: NM_002467.6), PGADT7::NMyc (NCBI accession number of Nmyc: NP_005369.2), PGADT7::c-Myc-150aa (the encoding gene sequence of c-Myc-150aa set forth in SEQ ID No.96, and the corresponding amino acid sequence set forth in SEQ ID No.95), and PGADT7::c-Myc-215aa (the encoding gene sequence of c-Myc-215aa set forth in SEQ ID No.98, and the corresponding amino acid sequence set forth in SEQ ID No.97) (the CDS of the above genes was subcloned to pGADT7(YouBio, VT1639) or pGBKT7(YouBio, VT16398) between the EcoRI and BamHI cleavage sites) were constructed using the competent Y2HGold for yeast two hybrid from Shanghai Weidi Company, followed by separate co-transfection of the yeast competent. The experiment was then carried out (the step of method as Example 3) according to the instructions of the competent cells to detect the interaction domain of cotton GhJAZ1 with uPAR and c-Myc.

The results from yeast two hybrid showed that the interaction of NT domain deficient GhJAZ1 with uPAR was significantly weakened (A in FIG. 13); and the interaction of D3 domain deficient uPAR with GhJAZ1 significantly weakened to none (B in FIG. 13), indicating that the NT domain of GhJAZ1 and the D3 domain of uPAR were involved in the interaction. The interaction of NT domain deficient GhJAZ1 with c-Myc was significantly weakened (C in FIG. 13); and the first 150 amino acids and 215 amino acids truncated c-Myc had strong interactions with GhJAZ1 (D and E in FIG. 13), indicating that the NT domain of GhJAZ1 interacted with the N-terminus of c-Myc.

### INDUSTRIAL APPLICATIONS

Cotton JAZ protein, plant JAZ homologous proteins and their derived polypeptides have significant inhibitory effects on tumor cell lines such as those of human breast cancer MCF7, ovarian cancer SKOV3, cervical cancer Hela, and lung cancer A549, indicating that JAZ protein and its derived polypeptides have a significant inhibitory effect on the occurrence and development of tumors in mice *in vivo.* Protein interaction experiments (yeast two hybrid, immunoprecipitation, pull-down experiment, bimolecular fluorescence complementarity, LUC experiment) confirmed strong interaction of JAZ protein and its derived polypeptides with human proto-oncogenes MYCs (c-Myc, NMYC, LMYC). In summary, JAZ protein and its derived polypeptides is capable of targeting various types of human and animal tumors and can be developed as targeting drugs against human and animal tumors. The present invention provides novel drugs or adjunctive drugs for the treatment of various human and animal tumors.

## Claims

1. Application of a), b), c) or d) in any one of the following P1-P4:
a). a plant JAZ protein;
b). a polypeptide derived from the plant JAZ protein;
c). a modified product of the plant JAZ protein in a) or the derived polypeptide in b); and
d). a relevant biomaterial of the plant JAZ protein in a) or the derived polypeptide in b); wherein the relevant biomaterial is any one of the followings: 1) a nucleic acid molecule encoding the plant JAZ protein or the derived polypeptides; 2) an expression cassette, an expression vector, or a recombinant microorganism comprising the nucleic acid molecule;
P1. preparation of products for the treatment and/or prevention of human or animals;
P2. preparation of products for inhibiting the occurrence and/or development of human or animal tumors;
P3. preparation of products for inhibiting the proliferation of human or animal tumor cells; and
P4. preparation of products for promoting apoptosis of human or animal tumor cells.

2. The application according to claim 1, wherein the plant JAZ protein is capable of interacting with MYC protein of humans or animals.

3. The application according to claim 2, wherein the MYC protein is c-Myc protein NMYC and/or LMYC protein.

4. The application according to any one of claims 1-3, wherein the plant JAZ protein has all or some of the following functions: arresting the cell cycle of a human or animal tumor cell at G2-M phase; regulating genes downstream of c-Myc in human or animal tumor cells; interacting with uPAR, a surface receptor for cancer cells; interacting with uPA, a ligand of the surface receptor uPAR for cancer cells; and entering into the nucleus of human or animal tumor cells.

5. The application according to claim 4, wherein the regulation of a gene downstream of c-Myc is embodied as all or some of the followings: apoptosis-related genes downstream of c-Myc, Bcl2, BAX, HK2, FASN, and MCM5 are down-regulated, and the P21 gene is up-regulated; and cell-cycle-related genes, CCNA2, CCNB1, and CCND1 are down-regulated, and CCNE1 is up-regulated.

6. The application according to any one of claims 1-5, wherein the plant JAZ protein is a JAZ protein derived from cotton.

7. The application according to claim 6, wherein the JAZ protein derived from cotton is any one of the followings:
(A1) a protein having an amino acid sequence as set forth in any one of SEQ ID No.1 to SEQ ID No.35;
(A2) a protein obtained by substitution and/or deletion and/or addition of one or more amino acid residues in the amino acid sequence defined in (A1) and derived from cotton having the same function;
(A3) a protein having more than 99%, more than 95%, more than 90%, more than 85%, or more than 80% identity to the amino acid sequence defined in any one of (A1) - (A2) and derived from cotton having the same function; and
(A4) a fusion protein obtained by linking a protein tag at the N-terminus and/or C-terminus of the protein defined in any one of (A1) - (A3).

8. The application according to claim 7, wherein the nucleic acid molecule encoding the plant JAZ protein is any one of the followings:
(B1) a DNA molecule as set forth in any one of SEQ ID No.36 to SEQ ID No.70;
(B2) a DNA molecule that hybridizes with the DNA molecule defined in (B1) under a strict condition and encodes the JAZ protein derived from cotton;
(B3) a DNA molecule having more than 99%, more than 95%, more than 90%, more than 85%, or more than 80% homology to the DNA sequence defined in any one of (B1) - (B2) and encoding the JAZ protein derived from cotton.

9. The application according to any one of claims 1-8, wherein the human or animal tumors are selected from the group consisting of ovarian, breast, cervical, and lung cancers; and the human or animal tumor cells are selected from the group consisting of ovarian, breast, cervical, and lung cancer cells.

10. The application according to claim 9, wherein the ovarian cancer cells are SKOV3 cells; the breast cancer cells are MCF7 cells; the cervical cancer cells are Hela cells; and the lung cancer cells are A549 cells.

11. The application according to any one of claims 1-10, wherein the product is a drug.

12. The application according to claim 11, wherein the drug is prepared by addition of pharmaceutically acceptable adjuvants on the basis of the a), b), or c).

13. The application according to claim 11 or 12, wherein the drug is a tablet, pill, granule, capsule, injection or oral solution.

14. Application of the a), b), c) or d) in any one of claims 1-8 in inhibiting the growth of plant callus.

15. The application according to claim 14, wherein the plant callus is cotton callus.

16. The application according to claim 14 or 15, wherein the plant JAZ protein has all or some of the following functions: interacting with NINJA protein, TPL protein, and HDA6 protein in a plant; interacting with COI1 protein in the presence of JA-Ile; and being capable of forming complexes with MYC2 protein, NINJA protein, and TPL protein.

17. Application of a JA synthesis inhibitor in inhibiting the growth of plant callus.

18. The application according to claim 17, wherein the JA synthesis inhibitor is DICEA that is a copper reagent or ibuprofen.

19. The application according to claim 17 or 18, wherein the plant callus is cotton callus.

20. A method for inhibiting the growth of plant callus the method is to inhibit the growth of plant callus with the a), b), c) or d) of any one of claims 1-8 or the JA synthesis inhibitors of claims 17 or 18.

21. The method according to claim 20, wherein the plant callus is cotton callus.

22. The method according to claim 20 or 21, wherein the method comprises the following steps of:
the experimental group: overexpressing the plant JAZ protein or the derived polypeptides of any one of claims 1-7 in the plant of interest to obtain a transgenic positive plant, taking an explant from the transgenic positive plant for tissue culture to obtain a callus;
the control group: the only difference from the experimental group is that the explant taken for tissue culture is from the plant of interest that is not transgenic; and
the growth rate of the callus in the experimental group is slower than that in the control group.

23. The method according to claim 20 or 21, wherein the method comprises the steps of:
the experimental group: placing an explant taken from a plant of interest in the callus induction medium supplemented with the JA synthesis inhibitor of claim 17 or 18 for tissue culture to obtain a callus;
the control group: the only difference from the experimental group is that the callus induction medium for tissue culture does not contain the JA synthesis inhibitor; and
the growth rate of the callus in the experimental group is slower than that in the control group.

24. A method for treating and/or preventing human or animal tumors, the method is to treat and/or prevent human or animal tumors using a drug with the a), b) or c) of any one of claims 1-8 as an active ingredient.

25. A method for inhibiting the occurrence and/or development of human or animal tumors, the method is to inhibit the occurrence and/or development of human or animal tumors using a drug with the a), b) or c) of any one of claims 1-8 as an active ingredient.

26. A method for inhibiting the proliferation of tumor cells in human or animal, the method is to inhibit the proliferation of human or animal tumor cells by the a), b) or c) of any one of claims 1-8.

27. A method for promoting apoptosis of tumor cells in human or animal, the method is to promote apoptosis of human or animal tumor cells by the a), b) or c) of any one of claims 1-8.

28. The method according to claim 26 or 27, wherein the concentration of the a) or b) or c) applied at a cellular level is controlled to be at more than 1 µM and less than 10 µM.

29. A drug having or containing the a), b), or c) of any one of claims 1-8 as an active ingredient, the drug is used for any one of the followings: treatment and/or prevention of human or animal tumors, inhibition of the occurrence and/or development of human or animal tumors, inhibition of the proliferation of human or animal tumor cells, and promotion of apoptosis of human or animal tumor cells.

30. A preparation having or containing the a), b), or c) of any one of claims 1-8 or the JA synthesis inhibitor of claim 17 or 18 as an active ingredient, which is used to inhibit the growth of plant callus.

31. The preparation according to claim 30, wherein the plant callus is cotton callus.
